# EUROPÄISCHE PATENTSCHRIFT

(11) **EP 0 954 267 B1**
(45) Veröffentlichungstag und Bekanntmachung des Hinweises auf die Patenterteilung: **09.04.2003**
(21) Anmeldenummer: 98907954.6
(22) Anmeldetag: 17.01.1998
(51) Int. Cl.: A61F 13/10, A61F 5/01

(54) **SELBSTKLEBENDE FERTIGBANDAGE ZUR IMMOBILISATION DES HANDGELENKS**
SELF-ADHESIVE READY-MADE BANDAGE FOR IMMOBILIZING THE WRIST
BANDAGE PREFORME POUR IMMOBILISER LE POIGNET

(30) Priorität: 23.01.1997 DE 19702300
(43) Veröffentlichungstag der Anmeldung: 10.11.1999
(73) Patentinhaber: Beiersdorf Aktiengesellschaft, 20245 Hamburg (DE)
(72) Erfinder: HIMMELSBACH, Peter, D-21614 Buxtehude (DE)
(86) Internationale Anmeldenummer: EP9800250
(87) Internationale Veröffentlichungsnummer: WO98032404

(56) Entgegenhaltungen:
- FR-A- 2 307 518
- US-A- 1 790 381
- US-A- 2 484 130
- US-A- 2 875 758
- US-A- 4 345 590

## Beschreibung

Die Erfindung betrifft eine einseitig selbstklebend beschichtete Fertigbandage zur Immobilisation des Handgelenks.

Die funktionelle Verbandtechnik, das sogenannte Taping, ist eine Behandlungsmethode zur Prophylaxe und Therapie von Verletzungen, Krankheiten und Veränderungen am Bewegungsapparat. Taping hat zum Ziel, die Kapsel-Band-Strukturen gezielt nachzubilden, und dadurch eine selektive Unterstützung und Stabilisierung zu erreichen.

Der eigentliche Tapeverband wird dabei streifenweise aus vorzugsweise unelastischen selbstklebenden Bändern, sogenannten Zügeln, oder in Verbindung mit kurzzugelastischen selbstklebenden Bändern angelegt. Er schützt, stützt und entlastet gefährdete, geschädigte oder gestörte Anteile einer Funktionseinheit. Er erlaubt die selektive Belastung im schmerzfreien Bewegungsraum, verhindert aber extreme oder schmerzhafte Bewegungen.

Das Anlegen derartiger Verbände erfordert jedoch fachmännisches Können und Erfahrung und kann deshalb in aller Regel nicht von Laien ohne Taping-Erfahrung ausgeführt werden.

Für das Handgelenk, das insbesondere bei sportlicher Betätigung oft sehr hohem mechanischen Streß ausgesetzt ist, der zu Distorsionen und Kontusionen, aber auch zu Zerrungen der Bänder im Handgelenk führen kann, verlangt der Fachmann aber eine einfach aufgebaute und gleichzeitig problemlos anzulegende Fertigbandage, die besonders bei leichteren Verletzungen einen positiven Einfluß auf den Heilungsprozeß nimmt.

Eine derartige Fertigbandage sollte aber auch der Laie anlegen können, so dass diesem eine preiswerte und wenig zeitintensive Hilfestellung durch die Fertigbandage geboten würde.

FR-A-2 307 518 offenbart eine Gelenkstützbandage für das Fuß-, Ellenbogen- und Handgelenk. Die Bandage weist eine "I" - Form auf mit zwei weiteren Befestigungslappen an jeder Längsseite, wobei beide Befestigungslappen seitlich direkt am Längsstreifen befestigt sind. Bei dieser offenbarten Ausführungsform einer Bandage für das Handgelenk ist ein Befestigungsflügel überflüssig und kann vom Anwender abgeschnitten werden. Weiterhin ist für die Anwendung am Handgelenk eine Öffnung für den Daumen vorgesehen.

Aufgabe der Erfindung war es deshalb, eine Fertigbandage zur Verfügung zu stellen, die aufgrund ihrer Ausgestaltung, ihres Materials und ihrer Eigenschaften zur Unterstützung des Heilungsprozesses bei Verletzungen im Handgelenk geeignet ist und die darüber hinaus vom Anwender in einfacher Weise angelegt werden kann.

Gelöst wird diese Aufgabe durch eine Fertigbandage gemäß Anspruch 1.

Demnach besteht die mindestens auf einer Seite selbstklebend beschichtete Fertigbandage zur Immobilisation des Handgelenks aus einem länglichen Streifen, an dem auf der einen Längsseite zumindest drei kurze Zügel angebracht sind und an dem an der gegenüberliegenden Längsseite ein zweiter Streifen angebracht ist, der mit dem länglichen Streifen einen Winkel zwischen 10° bis 150° einschließt.
An einer der Längsseiten des zweiten Streifens ist weiterhin ein dritter Streifen angebracht, der mit dem zweiten Streifen einen Winkel zwischen 30° bis 150° einschließt.

Vorzugsweise sind die kurzen Zügel in einem Winkel von 90° zum länglichen Streifen angebracht, wobei sich eine Zahl von fünf Zügel als besonders vorteilhaft erwiesen hat.

In einer weiteren bevorzugten Ausführungsform sind der längliche Streifen etwa 12 cm bis 30 cm lang und 2 cm bis 7 cm breit, der zweite Streifen etwa 5 bis 15 cm lang und 2 bis 6 cm breit, der dritte Streifen etwa 4 bis 12 cm lang und 2 cm bis 6 cm breit und die Zügel etwa 2 bis 10 cm lang und 2 cm bis 6 cm breit.
Die Abmessungen der Fertigbandage richten sich natürlich nach der Größe der Hand, an der die Fertigbandage angelegt wird. Für eine durchschnittliche Hand eines Erwachsenen weisen die einzelnen Teile der Fertigbandage die folgenden Maße auf:
- Der längliche Streifen ist 18 cm lang und 4 cm breit.
- Die zweite Streifen ist 10 cm lang und 4 cm breit.
- Die dritte Streifen ist 8 cm lang und 4 cm breit.
- Die Zügel sind 6 cm lang und 4 cm breit.

Alle Streifen und Zügel können darüber hinaus auch abgerundete Ecken aufweisen, um die Gefahr des ungewollten Ablösens der verklebten Fertigbandage zu verringern.

Als besonders vorteilhaft hat sich erwiesen, daß an den Stellen, an denen der längliche Streifen, der zweite Streifen, der dritte Streifen und/oder die Zügel zusammentreffen, Ausnehmungen oder Aussparungen vorhanden sind. Diese Ausnehmungen verhindern das Einreißen der Fertigbandage an den Punkten, die die höchste Beanspruchung insbesondere beim Anlegen aufweisen. Auf der anderen Seite erhöhen die Aussparungen die Flexibilität der Fertigbandage, so daß auf der anderen Seite das Anlegen erleichtert wird.

Weiterhin vorzugsweise besteht die erfindungsgemäße selbstklebende Fertigbandage aus einem unelastischen Gewebe oder Gewirke. Fallweise können sich auch elastische oder plastische Anteile in Längs- oder in Querrichtung des Trägermaterials vorteilhaft auf die Anwenderempfindung auswirken. Weiterhin können auch Vliesstoffe oder Schäume oder Papier eingesetzt werden, wenn diese eine ausreichende Festigkeit aufweisen.

Vorzugsweise kann das Trägermaterial aus Baumwolle bestehen, des weiteren eine Höchstzugkraft von nicht weniger als 50 N/cm und eine Höchstzugkraftdehnung von weniger als 20% aufweisen.

Die Fertigbandage ist auf der Seite, die auf der Haut aufgelegt wird, mit einer der bekannten gut haftenden Selbstklebemassen auf Basis von Kautschuk (vorzugsweise einer Zink-Kautschuk-Masse) oder synthetischen Polymeren beschichtet.
Bei der Selbstklebemasse kann es sich um Lösemittel-, Dispersions/Emulsionssysteme handeln, aber auch 100%- Selbstklebemassensysteme können Anwendung finden. Vorteilhaft weisen die Massen weitere Eigenschaften wie gute Hautverträglichkeit oder Luft- und Wasserdampfdurchlässigkeit auf.
Die Selbstklebemasse wird dabei mit ungefähr 100 g/m² aufgetragen.

Die Klebeschicht ist bis zum Gebrauch der Bandage mit einem klebstoffabweisend ausgerüstetem Blattmaterial wie beispielsweise silikonisiertem Papier, das darüber hinaus zur besseren Anwendung perforiert werden kann, oder einer Folie aus Kunststoff abgedeckt.

Das Material kann dabei in mehrere Abschnitte aufgegliedert sein, um das Anlegen der Fertigbandage durch sukzessives Ablösen der einzelnen Abschnitte zu erleichtern.

Die selbstklebende Fertigbandage ist universell zur Immobilisation des Handgelenks einsetzbar.

Selbstverständlich ist der Zuschnitt der Fertigbandage davon abhängig, an welcher Hand die Fertigbandage angelegt werden soll. Dementsprechend gibt es zwei spiegelsymmetrische Ausführungsformen der erfindungsgemäßen Fertigbandage.
Auch kann die Fertigbandage auf der palmaren oder der dorsalen Fläche der Hand angelegt werden, so daß der Zuschnitt je nach Anwendungsfall gesondert zu erfolgen hat.

Anhand der im folgenden beschriebenen Figur soll eine besonders vorteilhafte Ausführungsform der erfindungsgemäßen Fertigbandage sowie deren Anwendung näher dargestellt werden.

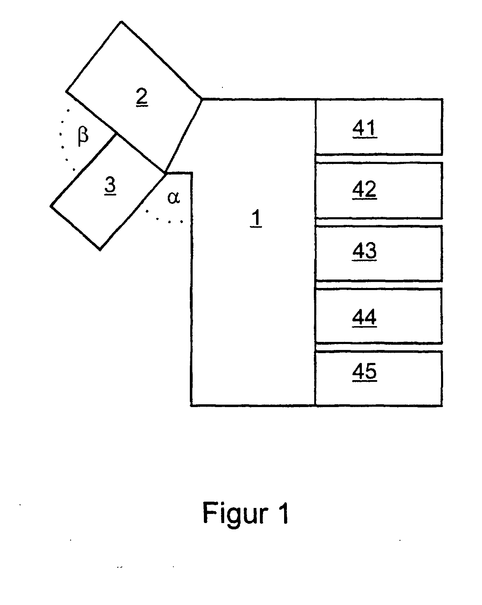

Die Figur 1 zeigt die Bandage in ihrer bevorzugten Ausführungsform. Die Fertigbandage setzt sich dabei aus mehreren Abschnitten zusammen. Der zentrale Abschnitt wird von einem länglichen Streifen (1) gebildet. An einer der Längsseiten des länglichen Streifens (1) schließen sich in einem rechten Winkel insgesamt fünf kurze, rechteckig geformte Zügel (41, 42, 43, 44, 45) an.
Auf der gegenüberliegenden Längsseite des länglichen Streifens (1), und zwar an der Kante zur Querseite, erweitert sich der längliche Streifen (1) in Form eines rechtwinkligen Dreiecks. An der Hypothenuse des genannten Dreiecks schließt sich der zweite Streifen (2) an, der ebenfalls eine annähernd rechteckige Form aufweist.
Weiterhin ist ein dritter, rechtwinklig geformter Streifen (3) so am zweiten Streifen (2) angeformt, daß sich der längliche Streifen (1), der zweite Streifen (2) und der dritte Streifen (3) in einem Punkt berühren.
Der Winkel α zwischen dem dritten Streifen (3) und dem länglichen Streifen (1) beträgt 45°, der Winkel β zwischen dem zweiten Streifen (2) und dem dritten Streifen (3) 90°.

Zum Anlegen der Bandage wird der längliche Streifen (1) palmar am Mittelfingeransatz angelegt, so daß der längliche Streifen (1) über das Handgelenk bis zum Unterarm verläuft. Der zweite Streifen (2) wird dann so verklebt, daß er von der Mittelhand zwischen Daumen und Zeigefinger zur dorsalen Fläche der Hand reicht. Der dritte Streifen (3) verläuft über den Daumenballen zur dorsalen Fläche der Hand.
Die fünf Zügel (41, 42, 43, 44, 45) werden abschließend ringförmig um die Hand, das Handgelenk beziehungsweise den Unterarm geführt.

Auf diese Weise ist die Bewegungsfähigkeit des Handgelenks annähernd bis zur vollständigen Immobilisation eingeschränkt. Eine noch stärkere Einschränkung läßt sich erzielen, in dem zusätzliche Zügel um die Hand, das Handgelenk beziehungsweise den Unterarm geklebt werden oder weitere Ankerstreifen den Verband stärken.

## Patentansprüche

1. Mindestens auf einer Seite selbstklebend beschichtete Fertigbandage zur Immobilisation des Handgelenks,
bestehend aus einem länglichen Streifen (1) mit zumindest drei kurzen Zügeln (41, 43, 45) an der einen Längsseite und
einem zweiten (2) und dritten (3) Streifen an der gegenüberliegenden Längsseite,
**dadurch gekennzeichnet, dass**
der zweite Streifen (2) mit dem länglichen Streifen (1) einen Winkel zwischen 10° bis 150° einschließt und dass
an einer der Längsseiten des zweiten Streifens (2) der dritte Streifen (3) angebracht ist, der mit dem zweiten Streifen (2) einen Winkel zwischen 30° bis 150° einschließt.

2. Selbstklebende Fertigbandage gemäß Anspruch 1, **dadurch gekennzeichnet, daß** die Zügel (41, 43, 45) in einem Winkel von 90° zum länglichen Streifen angebracht sind.

3. Selbstklebende Fertigbandage gemäß einem der Ansprüche 1 oder 2, **dadurch gekennzeichnet, daß** an dem länglichen Streifen (1) fünf Zügel (41, 42, 43, 44, 45) vorhanden sind.

4. Selbstklebende Fertigbandage gemäß einem der Ansprüche 1 bis 3, **dadurch gekennzeichnet, daß**
der längliche Streifen (1) etwa 12 bis 30 cm, insbesondere 18 cm, lang und 2 cm bis 7 cm, insbesondere 4 cm, breit ist,
der zweite Streifen (2) etwa 5 cm bis 15 cm, insbesondere 10 cm, lang und 2 cm bis 6 cm, insbesondere 4 cm, breit ist,
der dritte Streifer (3) etwa 4 cm bis 12 cm, insbesondere 8 cm, lang und 2 cm bis 6 cm, insbesondere 4 cm, breit ist,
die Zügel (41, 42, 43, 44, 45) etwa 2 bis 10 cm, insbesondere 6 cm, lang und 2 cm bis 6 cm, insbesondere 4 cm, breit sind.

5. Selbstklebende Fertigbandage gemäß einem der Ansprüche 1 bis 4, **dadurch gekennzeichnet, daß** die Zügel (41, 42, 43, 44, 45) identische Abmessungen aufweisen.

6. Selbstklebende Fertigbandage gemäß einem der Ansprüche 1 bis 5, **dadurch gekennzeichnet, daß** an den Stellen, an denen der längliche Streifen (1), der zweite Streifen (2), der dritte Streifen (3) und/oder die Zügel (41, 42, 43, 44, 45) zusammentreffen, Ausnehmungen vorhanden sind.

7. Selbstklebende Fertigbandage gemäß einem der Ansprüche 1 bis 6, **dadurch gekennzeichnet, daß** die Fertigbandage aus einem unelastischem Trägermaterial besteht, insbesondere Vliese, Papiere, Schäume, Gewebe oder Gewirke.

8. Selbstklebende Fertigbandage gemäß einem der Ansprüche 1 bis 7, **dadurch gekennzeichnet, daß** das Gewebe aus Baumwolle besteht und eine Höchstzugkraft von nicht weniger als 50 N/cm und eine Höchstzugkraftdehnung von weniger als 20% aufweist.

9. Selbstklebende Fertigbandage gemäß einem der Ansprüche 1 bis 8, **dadurch gekennzeichnet, daß** die Fertigbandage auf ihrer selbstklebenden Seite mit klebstoffabweisendem Material abgedeckt ist.

10. Selbstklebende Fertigbandage gemäß einem der Ansprüche 1 bis 9, **dadurch gekennzeichnet, daß** die Fertigbandage universell zur Immobilisation des Handgelenks einsetzbar ist.

## Claims

1. Ready-made bandage with a self-adhesive coating on at least one side, for immobilizing the wrist, consisting of an oblong strip (1) with at least three short reins (41, 43, 45) on one long side, and a second (2) and third (3) strip on the opposite long side, **characterized in that** the second strip (2) encloses an angle of between 10° and 150° with the oblong strip (1), and **in that** the third strip (3) is arranged on one of the long sides of the second strip (2) and encloses an angle of between 30° and 150° with the second strip (2).

2. Self-adhesive ready-made bandage according to Claim 1, **characterized in that** the reins (41, 43, 45) are arranged at an angle of 90° to the oblong strip.

3. Self-adhesive ready-made bandage according to one of Claims 1 or 2, **characterized in that** five reins (41, 42, 43, 44, 45) are present on the oblong strip (1).

4. Self-adhesive ready-made bandage according to one of Claims 1 to 3, **characterized in that** the oblong strip (1) is about 12 to 30 cm long, in particular 18 cm, and 2 cm to 7 cm wide, in particular 4 cm, the second strip (2) is about 5 cm to 15 cm long, in particular 10 cm, and 2 cm to 6 cm wide, in particular 4 cm, the third strip (3) is about 4 cm to 12 cm long, in particular 8 cm, and 2 cm to 6 cm wide, in particular 4 cm, and the reins (41, 42, 43, 44, 45) are about 2 to 10 cm long, in particular 6 cm, and 2 cm to 6 cm wide, in particular 4 cm.

5. Self-adhesive ready-made bandage according to one of Claims 1 to 4, **characterized in that** the reins (41, 42, 43, 44, 45) have identical dimensions.

6. Self-adhesive ready-made bandage according to one of Claims 1 to 5, **characterized in that** cuttings are present at the points where the oblong strip (1), the second strip (2), the third strip (3) and/or the reins (41, 42, 43, 44, 45) meet.

7. Self-adhesive ready-made bandage according to one of Claims 1 to 6, **characterized in that** the ready-made bandage consists of a non-elastic support material, in particular nonwovens, papers, foams, woven fabrics or knitted fabrics.

8. Self-adhesive ready-made bandage according to one of Claims 1 to 7, **characterized in that** the woven fabric consists of cotton and has a maximum tensile force of not less than 50 N/cm and a maximum tensile force extension of less than 20%.

9. Self-adhesive ready-made bandage according to one of Claims 1 to 8, **characterized in that** the ready-made bandage is covered, on its self-adhesive side, with anti-adhesive material.

10. Self-adhesive ready-made bandage according to one of Claims 1 to 9, **characterized in that** the ready-made bandage can be used universally for immobilizing the wrist.

## Revendications

1. Bandage prêt à l'emploi revêtu au moins d'un côté de manière auto-adhésive pour l'immobilisation du poignet, constitué d'une bande longitudinale (1) présentant au moins trois courtes brides (41, 43, 45) sur l'un des côtés longitudinaux et une deuxième (2) et une troisième bande (3) sur le côté longitudinal opposé, **caractérisé en ce que** la deuxième bande (3) forme avec la bande longitudinale (1) un angle entre 10° et 150° et que, sur l'un des côtés longitudinaux de la deuxième bande (2), la troisième bande (3) est appliquée, qui forme avec la deuxième bande (2) un angle entre 30° et 150°.

2. Bandage auto-adhésif prêt à l'emploi selon la revendication 1, **caractérisé en ce que** les brides (41, 43, 45) sont placées dans un angle de 90° par rapport à la bande longitudinale.

3. Bandage auto-adhésif prêt à l'emploi selon l'une quelconque des revendications 1 ou 2, **caractérisé en ce que** cinq brides (41, 42, 43, 44, 45) se situent au niveau de la bande longitudinale (1).

4. Bandage auto-adhésif prêt à l'emploi selon l'une quelconque des revendications 1 à 3, **caractérisé en ce que** la bande longitudinale (1) présente une longueur d'environ 12 à 30 cm, en particulier de 18 cm, et une largeur de 2 à 7 cm, en particulier de 4 cm, la deuxième bande (2) présente une longueur d'environ 5 à 15 cm, en particulier de 10 cm, et une largeur de 2 à 6 cm, en particulier de 4 cm, la troisième bande (3) présente une longueur d'environ 4 à 12 cm, en particulier de 8 cm, et une largeur de 2 à 6 cm, en particulier de 4 cm, et les brides (41, 42, 43, 44, 45) présentent une longueur d'environ 2 à 10 cm, en particulier de 6 cm, et une largeur de 2 à 6 cm, en particulier de 4 cm.

5. Bandage auto-adhésif prêt à l'emploi selon l'une quelconque des revendications 1 à 4, **caractérisé en ce que** les brides (41, 42, 43, 44, 45) présentent des dimensions identiques.

6. Bandage auto-adhésif prêt à l'emploi selon l'une quelconque des revendications 1 à 5, **caractérisé en ce qu'**on a prévu des évidements aux endroits où la bande longitudinale (1), la deuxième bande (2), la troisième bande (3) et/ou les brides (41, 42, 43, 44, 45) se rencontrent.

7. Bandage auto-adhésif prêt à l'emploi selon l'une quelconque des revendications 1 à 6, **caractérisé en ce que** le bandage prêt à l'emploi est constitué d'un matériau support inélastique, en particulier des non-tissés, des papiers, des mousses, des tissus ou des étoffes.

8. Bandage auto-adhésif prêt à l'emploi selon l'une quelconque des revendications 1 à 7, **caractérisé en ce que** le tissu est constitué de coton et présente une résistance maximale à la traction non inférieure à 50 N/cm et un allongement maximal à la traction inférieur à 20%

9. Bandage auto-adhésif prêt à l'emploi selon l'une quelconque des revendications 1 à 8, **caractérisé en ce que** le bandage prêt à l'emploi est recouvert sur sa face auto-adhésive d'un matériau répulsif à l'adhésif.

10. Bandage auto-adhésif prêt à l'emploi selon l'une quelconque des revendications 1 à 9, **caractérisé en ce que** le bandage prêt à l'emploi peut être utilisé universellement pour l'immobilisation du poignet.
